(19) Europäisches Patentamt
European Patent Office
Office européen des brevets

(11) **EP 3 210 531 B1**

(12) **FASCICULE DE BREVET EUROPEEN**

(45) Date de publication et mention
de la délivrance du brevet:
**16.02.2022 Bulletin 2022/07**

(21) Numéro de dépôt: **17158181.2**

(22) Date de dépôt: **27.02.2017**

(51) Classification Internationale des Brevets (IPC):
**A61B 5/024** *(2006.01)*     **A61B 5/1455** *(2006.01)*
**A61B 5/00** *(2006.01)*

(52) Classification Coopérative des Brevets (CPC):
**A61B 5/02433; A61B 5/14552; A61B 5/7214;**
A61B 5/7282; A61B 2562/0238

(54) **PROCÉDÉ D'ESTIMATION D'UNE FRÉQUENCE CARDIAQUE ET DISPOSITIF ASSOCIÉ**

VERFAHREN ZUR ABSCHÄTZUNG DER HERZFREQUENZ, UND ENTSPRECHENDE VORRICHTUNG

METHOD FOR ESTIMATING A CARDIAC FREQUENCY, AND ASSOCIATED DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.02.2016 FR 1651656**

(43) Date de publication de la demande:
**30.08.2017 Bulletin 2017/35**

(73) Titulaire: **COMMISSARIAT À L'ÉNERGIE ATOMIQUE ET AUX ÉNERGIES ALTERNATIVES**
**75015 Paris (FR)**

(72) Inventeurs:
• **STANCHINA, Sylvain**
  **38000 Grenoble (FR)**
• **KOENIG, Anne**
  **38410 Saint Martin d'Uriage (FR)**

(74) Mandataire: **INNOV-GROUP**
  **310, avenue Berthelot**
  **69372 Lyon Cedex 08 (FR)**

(56) Documents cités:
**US-A1- 2013 072 771    US-A1- 2015 196 257**

**Description**

## DOMAINE TECHNIQUE

Le domaine technique de l'invention est la mesure optique d'une fréquence cardiaque d'un individu ou d'un animal.

## ART ANTERIEUR

Les mesures optiques sont fréquemment mises en oeuvre pour déterminer des paramètres physiologiques d'un être vivant, en particulier liés au sang. Des dispositifs, dits oxymètres de pouls, sont couramment utilisés pour déterminer une saturation en oxygène de l'hémoglobine dans le sang. Ces mesures sont basées sur l'absorption de la lumière par l'hémoglobine, cette dernière variant entre l'oxyhémoglobine et la déoxyhémoglobine. Des dispositifs commerciaux sont largement utilisés, basés sur une mesure de la lumière transmise par un organe suffisamment fin, dans une bande spectrale infrarouge et une bande spectrale rouge. L'organe examiné peut notamment être l'extrémité d'un doigt ou le lobe d'une oreille. Les mesures de la lumière transmise, dans chacune des bandes spectrales, permettent d'estimer des concentrations en oxyhémoglobine et déoxyhémoglobine dans le sang, à partir desquelles on estime la saturation en oxygène du sang. Ces mesures permettent également de détecter un flux pulsatile de sang et d'en déduire une fréquence cardiaque. La plupart des oxymètres commerciaux permettent également une estimation de la fréquence cardiaque à partir des mesures réalisées dans l'une ou l'autre bande spectrale.

Le brevet européen EP2355693 décrit par exemple un dispositif comportant une première source de lumière émettant dans une longueur d'onde rouge et une deuxième source de lumière émettant dans une longueur d'onde infrarouge. Un photodétecteur est apte à détecter un rayonnement lumineux émanant d'un doigt éclairé par une desdites sources de lumière. La source de lumière infra-rouge est notamment utilisée pour détecter la présence du doigt contre le dispositif, suite à quoi la source de lumière rouge est activée, de façon à permettre une détermination de paramètres tels la fréquence cardiaque ou l'oxymétrie de pouls.

Le brevet américain US9042971 décrit un dispositif nomade d'actimétrie, permettant de déterminer de façon optique une fréquence cardiaque par une détection d'un rayonnement lumineux rétrodiffusé par un doigt sous l'effet d'une illumination. Ce dispositif permet d'effectuer des mesures selon une configuration de rétrodiffusion, également désignée par le terme configuration en réflectance, les sources de lumière étant disposées de façon adjacente au photodétecteur.

Le document US2013/0072771 décrit un dispositif pour illuminer le doigt d'un utilisateur selon deux longueurs d'onde différentes. Des images du rayonnement rétrodiffusé par le doigt sont acquises à chaque longueur d'onde, à partir desquelles une fréquence cardiaque de l'utilisateur est calculée.

Les inventeurs ont constaté que de tels dispositifs pouvaient générer des erreurs de mesure de la fréquence cardiaque, notamment lorsqu'ils sont portés par une personne en mouvement. L'invention propose de résoudre ce problème, en permettant d'obtenir des mesures plus fiables de la fréquence cardiaque.

## EXPOSE DE L'INVENTION

Un premier objet de l'invention est un procédé d'estimation d'une fréquence cardiaque d'un être vivant comportant les étapes suivantes :

a) illumination d'une zone corporelle dudit être vivant par un faisceau lumineux incident selon une première bande spectrale ;
b) détection d'un rayonnement lumineux transmis ou rétrodiffusé, dans ladite bande spectrale, par ladite zone corporelle sous l'effet de ladite illumination ;
c) détermination d'une première fonction de détection, représentant une évolution temporelle d'une intensité du rayonnement lumineux ainsi détecté ;
d) identification d'instants caractéristiques à partir de ladite première fonction de détection, et calcul d'une fréquence d'occurrence desdits instants caractéristiques , chaque instant caractéristique correspondant à un instant auquel ladite fonction de détection atteint une valeur particulière, ou franchit un seuil, ou atteint un extremum local ;
e) estimation d'une fréquence cardiaque à partir de ladite fréquence d'occurrence ; où :

- les étapes a) à b) sont également mises en œuvre dans une deuxième bande spectrale, de préférence différente de la première bande spectrale, de telle sorte que l'étape c) comporte une détermination d'une deuxième fonction de détection représentant une évolution temporelle d'une intensité du rayonnement lumineux détecté dans la deuxième bande spectrale ;

- l'étape d) comporte une identification d'instants caractéristiques à partir de la deuxième fonction de détection ainsi qu'une sélection d'instants caractéristiques identifiés à partir de chaque fonction de détection, et apparaissant en coïncidence temporelle, la fréquence d'occurrence étant calculée à partir des instants caractéristiques ainsi sélectionnés.

L'étape d) peut comporter un calcul d'une première fonction dérivée à partir de la première fonction de détection, et d'une deuxième fonction dérivée à partir de la deuxième fonction de détection, ainsi que l'identification d'instants caractéristiques à partir de chacune desdites fonctions dérivées. Chaque fonction dérivée peut être obtenue par une différence entre la valeur d'une fonction de détection en deux instants différents.

Par en coïncidence temporelle, on entend simultanément, c'est-à-dire dans une même fenêtre temporelle. Cette fenêtre temporelle peut être prédéterminée où ajustable. Lorsqu'un instant caractéristique est identifié à partir de la première fonction de détection et qu'aucun instant caractéristique n'est identifié, en coïncidence temporelle, à partir de la deuxième fonction de détection, l'instant caractéristique identifié à partir de la première fonction de détection est invalidé et n'est pas pris en compte dans la détermination de la fréquence d'occurrence.

Par faisceau lumineux ou rayonnement lumineux, on entend un flux de photons dont la bande spectrale est comprise dans le domaine visible, ou proche infra-rouge, ou proche UV, par exemple entre 200 nm et 1000 nm.

La fréquence cardiaque peut être égale à la fréquence d'occurrence.

Le procédé peut comporter l'une des caractéristiques suivantes, prises isolément ou en combinaison :

- la première bande spectrale comporte des longueurs d'onde comprises entre 600 et 700 nm ;
- la deuxième bande spectrale comporte des longueurs d'onde comprises entre 750 nm et 1 $\mu$m ;
- la première bande spectrale s'étend entre 600 nm et 700 nm, et/ou la deuxième bande spectrale s'étend entre 750 nm et 1 $\mu$m, et de préférence entre 810 nm et 1000 nm ;
- la première bande spectrale s'étend, majoritairement ou en totalité, en dessous de 805 nm, tandis que la deuxième bande spectrale s'étend, majoritairement ou en totalité, en dessus de 805 nm.

Le rayonnement détecté peut être un rayonnement rétrodiffusé, auquel cas la source de lumière et le photodétecteur sont disposés de façon adjacente l'un par rapport à l'autre. Le rayonnement détecté peut également être un rayonnement transmis, auquel cas la zone corporelle s'étend entre la source de lumière et le photodétecteur.

De préférence, la première bande spectrale et la deuxième bande spectrale ne se recouvrent pas, où de façon négligeable.

Un autre objet de l'invention est un dispositif pour l'estimation d'une fréquence cardiaque d'un être vivant, comportant :

- une source de lumière apte à émettre un faisceau lumineux incident se propageant vers une zone corporelle dudit être vivant, selon une première bande spectrale et selon une deuxième bande spectrale ;
- un photodétecteur, apte à détecter, dans chaque bande spectrale, un rayonnement rétrodiffusé ou transmis par ladite zone corporelle, sous l'effet de son illumination par ledit faisceau lumineux incident ;
- un processeur, configuré pour mettre en œuvre les étapes c) à e) du procédé décrit dans cette demande.

Le photodétecteur peut être apte à détecter un rayonnement rétrodiffusé par ladite zone corporelle sous l'effet de son illumination. Alternativement, le photodétecteur est apte à détecter un rayonnement transmis par la zone corporelle illuminée.

La source de lumière peut comporter :

- une première source de lumière élémentaire, apte à émettre un premier faisceau lumineux incident selon la première bande spectrale;
- une deuxième source de lumière élémentaire, apte à émettre un deuxième faisceau lumineux incident selon la deuxième bande spectrale.

D'autres avantages et caractéristiques ressortiront plus clairement de la description qui va suivre de modes particuliers de réalisation de l'invention, donnés à titre d'exemples non limitatifs, et représentés aux figures annexées, listées ci-dessous.

## FIGURES

Les figures 1A et 1B représentent respectivement un premier et un deuxième exemple de dispositif apte à mettre en œuvre l'invention.

La figure 2A représente une fonction de détection obtenue suite à la détection d'un rayonnement rétrodiffusé d'une zone corporelle sous l'effet d'une illumination.

La figure 2B représente une fonction dite dérivée, obtenue à partir d'une fonction de détection telle que représentée sur la figure 2A.

La figure 3A représente un détail d'une fonction de détection, et de sa fonction dérivée, en fonction du temps. Elle illustre la survenue d'un front intempestif.

La figure 3B représente des estimations, en fonction du temps, de la fréquence cardiaque d'un individu en fonction d'un signal détecté en réponse à une illumination selon une bande spectrale respectivement centrée sur 660 nm et 940 nm. Ces estimations sont réalisées selon un procédé de l'art antérieur et ont été entreprises en illuminant le pouce d'un individu, selon une configuration en rétrodiffusion.

La figure 4A représente les principales étapes d'un procédé selon l'invention. La figure 4B illustre chaque séquence d'éclairement du procédé.

Les figures 5A et 5B représentent respectivement la détection d'instants caractéristiques à partir d'une première fonction de détection et d'une deuxième fonction de détection.

La figure 6A est identique à la figure 3B. La figure 6B représente une évaluation de la fréquence cardiaque d'un individu, en mettant en œuvre l'invention, en fonction des signaux détectés pour établir la figure 6A.

La figure 6C correspond à une évaluation de la fréquence cardiaque d'un individu, réalisée selon un procédé de l'art antérieur, en illuminant le poignet d'un individu. La figure 6D correspond à une évaluation de la fréquence cardiaque, en mettant en œuvre l'invention, en fonction des signaux détectés pour établir la figure 6C.

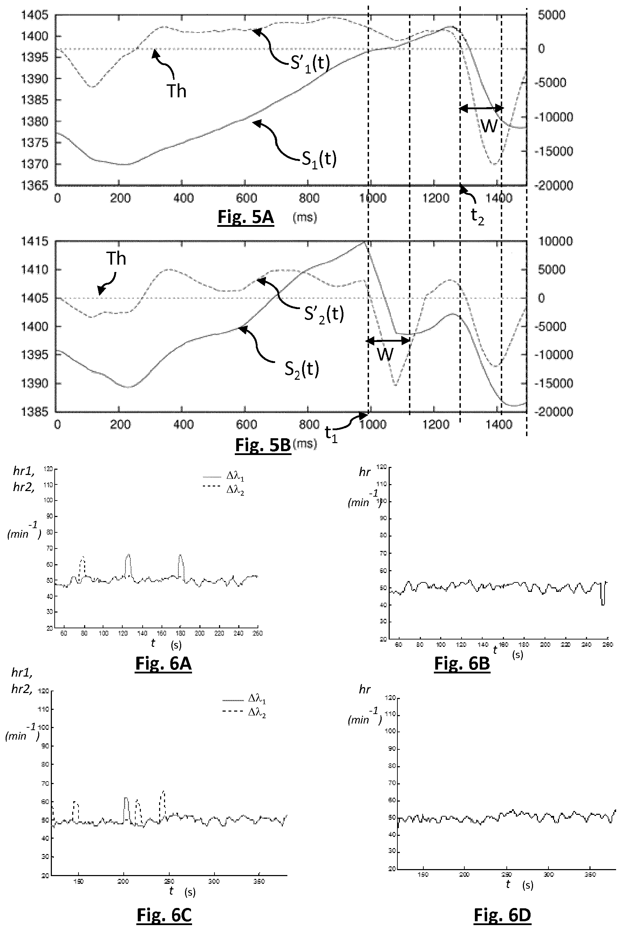

Les figures 6A à 6D sont réalisées en mettant en œuvre le procédé selon une configuration en rétrodiffusion.

## EXPOSE DE MODES DE REALISATION PARTICULIERS

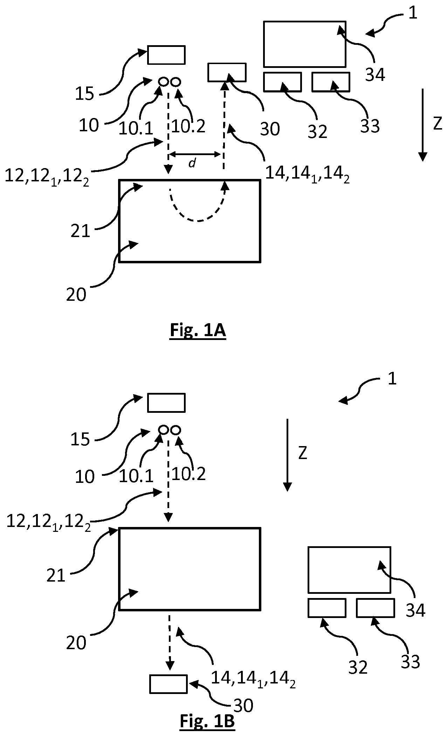

La figure 1A représente un exemple d'un dispositif 1 permettant la mise en œuvre de l'invention. Une source de lumière 10 émet un faisceau lumineux incident 12 se propageant jusqu'à un échantillon 20, selon un axe de propagation Z. Le terme échantillon désigne une zone corporelle d'un être vivant, dont on souhaite acquérir la fréquence cardiaque. Les photons composant le faisceau lumineux incident 12 pénètrent dans l'échantillon et une partie d'entre eux est rétrodiffusée selon une direction parallèle à l'axe de propagation, dans un sens opposé à ce dernier. Ces photons rétrodiffusés constituent un rayonnement rétrodiffusé 14. Le rayonnement rétrodiffusé 14 peut être détecté par un photodétecteur 30, placé en regard de la surface 21 de l'échantillon. Le photodétecteur peut être configuré de façon à détecter un rayonnement rétrodiffusé émanant de l'échantillon à une distance $d$, dite distance de rétrodiffusion, généralement non nulle et inférieure à quelques millimètres, typiquement inférieure à 15 mm ou 10 mm.

Dans cet exemple, la source de lumière 10 comporte deux sources de lumières élémentaires $10_1$ et $10_2$. La première source de lumière élémentaire $10_1$ est une diode électroluminescente émettant dans une première bande spectrale $\Delta\lambda_1$ centrée sur une première longueur d'onde $\lambda_1$ égale à 660 nm. Il s'agit d'une diode électroluminescente fournie par le fabricant Kingbright sous la référence APT1608SURCK. La deuxième source de lumière élémentaire $10_2$ est une diode électroluminescente émettant dans une deuxième bande spectrale $\Delta\lambda_2$ centrée sur une deuxième longueur d'onde $\lambda_2$ égale à 940 nm. Il s'agit d'une diode électroluminescente fournie par le fabricant Kingbright sous la référence APT1608F3C. Ainsi, la première bande spectrale $\Delta\lambda_1$ s'étend de préférence entre 600 et 700 nm, ce qui recouvre la bande spectrale visible rouge, tandis que la deuxième bande spectrale $\Delta\lambda_2$ s'étend de préférence entre 700 et 1000 nm, et encore de préférence entre 810 nm - 1000 nm, ce qui correspond à une bande spectrale dans le proche infrarouge. De préférence, la première bande spectrale $\Delta\lambda_1$ et la deuxième bande spectrale $\Delta\lambda_2$ sont différentes et ne se recouvrent pas. Par ne pas se recouvrir, on entend que la majeure partie du spectre d'émission, et de préférence 80% voire plus de 90% de l'intensité émise, ne se situe pas dans la même plage spectrale.

Un microcontrôleur 15 pilote l'activation séquentielle des sources de lumières élémentaires $10_1$ et $10_2$. Ainsi, l'échantillon est successivement illuminé par un premier faisceau lumineux incident $12_1$, dans la première bande spectrale $\Delta\lambda_1$, et par un deuxième faisceau lumineux incident $12_2$, dans la deuxième bande spectrale $\Delta\lambda_2$.

Un photodétecteur 30 détecte un premier rayonnement rétrodiffusé $14_1$, dans la première bande spectrale $\Delta\lambda_1$, sous l'effet de l'illumination par le premier faisceau lumineux incident $12_1$, ainsi qu'un deuxième rayonnement rétrodiffusé $14_2$, dans la deuxième bande spectrale $\Delta\lambda_2$ sous l'effet de l'illumination par le deuxième faisceau lumineux incident $12_2$. Dans l'exemple représenté, le photodétecteur est une photodiode fournie par VISHAY sous la référence BPW345, dont la bande spectrale de détection permet la détection du premier et du deuxième rayonnement rétrodiffusé. La distance de rétrodiffusion $d$ s'élève, dans cet exemple, à 7 mm.

Un processeur 32 est apte à établir une fonction de détection, correspondant à une évolution temporelle de l'intensité d'un rayonnement détecté par le photodétecteur, dans chacune des bandes spectrales. Il peut être relié à une mémoire 33, apte à stocker des instructions permettant de mettre en oeuvre un procédé décrit dans cette description.

Il peut également être relié à une unité d'affichage 34.

Selon un autre mode de réalisation, représenté sur la figure 1B, le dispositif 1 est disposé selon une configuration dite en transmission. Dans une telle configuration, l'échantillon s'étend entre la source de lumière 10 et le photodétecteur 30. Ce dernier est alors apte à détecter un rayonnement 14 transmis par l'échantillon selon l'axe de propagation Z. Cependant, un tel dispositif est moins compact que le dispositif en rétrodiffusion exposé en lien avec la figure 1A. De plus, un dispositif en rétrodiffusion, tel que présenté sur la figure 1A, est plus versatile qu'un dispositif fonctionnant en transmission. En effet, son fonctionnement est indépendant de l'épaisseur de la zone corporelle analysée. Il peut donc être placé contre différentes zones corporelles, par exemple un poignet, un doigt, ou une jambe.

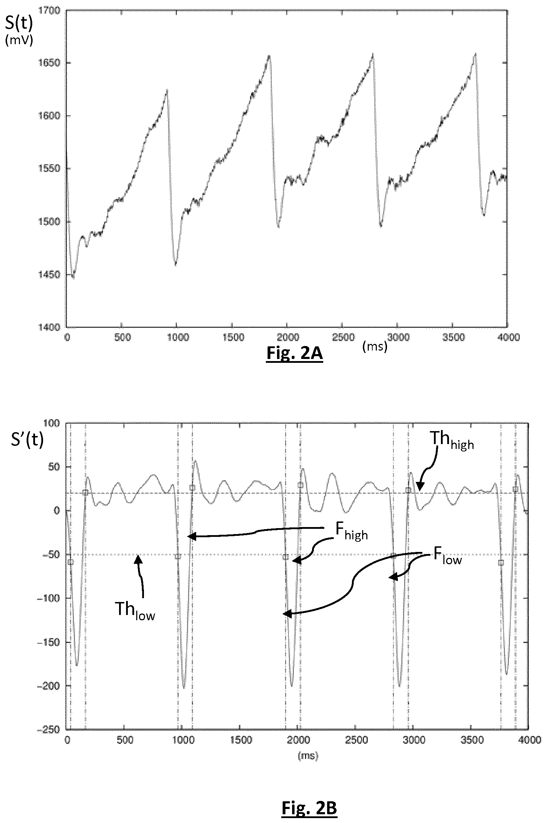

La figure 2A représente une fonction S(t), dite fonction de détection, représentant l'intensité, S, en fonction du temps, d'un rayonnement rétrodiffusé (ou transmis) détecté par le photodétecteur 30, dans la première bande spectrale. Sous l'effet de l'activité cardiaque, l'intensité du rayonnement rétrodiffusé suit une évolution temporelle périodique, dont la période est liée à la fréquence cardiaque $hr$. La figure 2B représente l'évolution, en fonction du temps, d'une fonction, dite dérivée, représentant une dérivée temporelle de la fonction de détection. On comprend qu'il est aisé d'estimer la fréquence de la fonction S'(t), cette dernière correspondant aussi à la fréquence de la fonction S(t), qui correspond également à la fréquence cardiaque hr recherchée. Une méthode connue de l'art antérieur consiste à utiliser un seuil bas $Th_{low}$ et un seuil haut $Th_{high}$, de façon à détecter respectivement un front descendant $F_{low}$ et un front montant $F_{high}$ dans la fonction dérivée S'(t). Les instants correspondant à un front descendant, ou à un front montant, sont appelés instants caractéristiques. Ils sont respectivement obtenus en déterminant les instants auxquels la fonction dérivée S'(t) franchit le seuil bas et le seuil haut, à une période d'échantillonnage près. La fréquence $f$ de la fonction dérivée est obtenue en calculant une période entre deux instants caractéristiques successifs, ou par une moyenne de l'intervalle temporel entre plusieurs instants caractéristiques consécutifs.

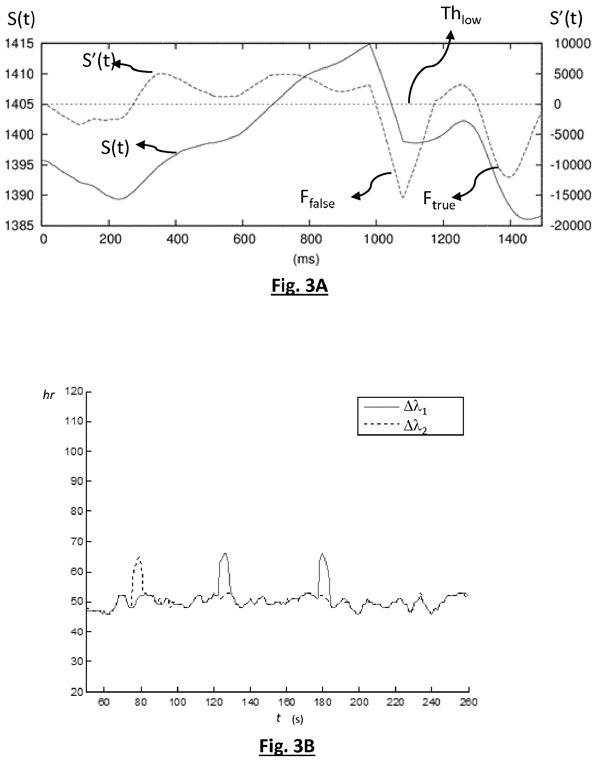

Une telle estimation est généralement effectuée dans une seule bande spectrale, qu'il s'agisse d'une bande spectrale rouge ou d'une bande spectrale infra-rouge. Cependant, ce type d'estimation manque de robustesse. Plus particulièrement, des mouvements de la zone corporelle illuminée, des expositions à des sources de lumière parasites ou de simples bruits électroniques peuvent fausser l'estimation de la fréquence cardiaque. La figure 3A représente un détail d'une fonction de détection et d'une fonction dérivée dans un intervalle temporel de 1500 ms. Cette figure illustre un exemple de détection d'un front descendant, dit intempestif $F_{false}$, et d'un front descendant $F_{true}$, dit réel. Les inventeurs ont estimé qu'il était nécessaire de réduire la détection de fronts intempestifs, car leur présence fausse l'estimation de la fréquence cardiaque.

La figure 3B représente des estimations de la fréquence cardiaque $hr_1, hr_2$ calculées respectivement à partir d'une première fonction de détection $S_1(t)$ obtenue à partir d'une illumination d'un échantillon dans la première bande spectrale $\Delta\lambda_1$, et d'une deuxième fonction de détection $S_2(t)$ obtenue à partir d'une illumination de l'échantillon dans la deuxième bande spectrale $\Delta\lambda_2$. Sur chacune de ces estimations, on observe des variations intempestives des estimations, dues à des détections de fronts intempestifs tels que le front $F_{false}$ décrit en lien avec la figure 3A.

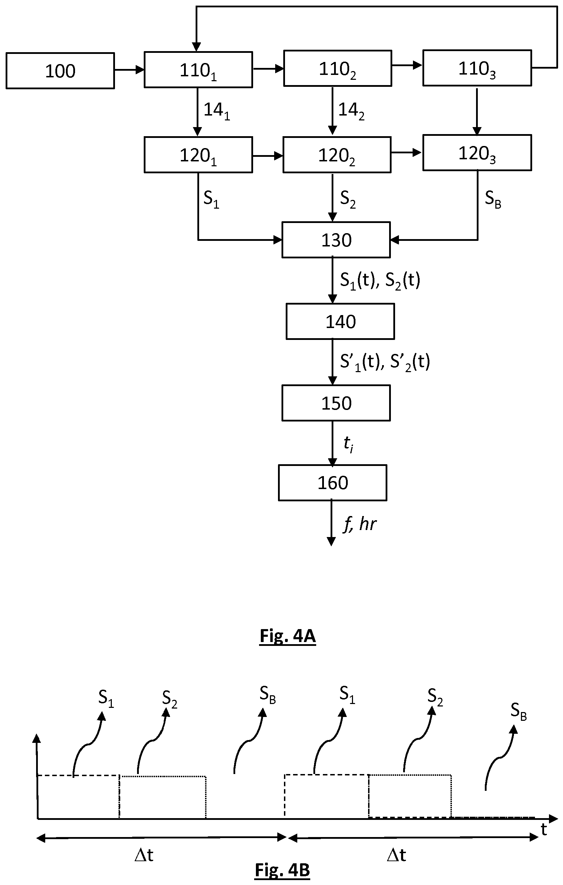

Les inventeurs ont défini un procédé permettant d'éviter la détection d'instants caractéristiques intempestifs. Ce procédé, combinant la détection de signaux rétrodiffusés ou transmis dans deux bandes spectrales, est défini ci-dessous, en lien avec la figure 4A, dont les principales étapes sont présentées ci-après.

Etape 100 : disposition du dispositif 1, de telle sorte que la source de lumière 10 est apte à illuminer un échantillon, c'est-à-dire une zone corporelle 20 d'un être vivant, et que le photodétecteur est apte à détecter un rayonnement rétrodiffusé ou transmis par ladite zone corporelle consécutivement à cette illumination.

Etape 110 : illumination de l'échantillon 20 dans la première bande spectrale $\Delta\lambda_1$ (sous-étape $110_1$) et dans la deuxième bande spectrale $\Delta\lambda_2$ (sous-étape $110_2$). En fonction du photodétecteur utilisé, cette illumination peut être simultanée ou successive. Dans cet exemple, on utilise un unique photodétecteur non résolu spectralement. L'échantillon est illuminé successivement par chaque source élémentaire $10_1$ et $10_2$, la durée de chaque illumination étant de 1,66 ms. L'activation successive de chaque source de lumière élémentaire, désignée par le terme « séquence d'éclairement » est commandée par le microcontrôleur 15. Alternativement, les sources de lumière peuvent être activées en continu, le rayonnement rétrodiffusé (ou transmis) étant détecté par deux photodétecteurs différents, chacun étant apte à détecter respectivement ledit rayonnement dans ladite première bande spectrale ou ladite deuxième bande spectrale. Selon une autre variante, le photodétecteur peut être spectralement résolu, ce qui permet également une illumination de la zone corporelle 20 simultanément selon les deux bandes spectrales. De façon préférée, mais optionnelle, après l'activation de la deuxième source de lumière, aucune source lumineuse n'est activée pendant 1,66 ms (sous-étape $110_3$). Le signal $S_B$ détecté par le photodétecteur 30 est alors représentatif d'un courant d'obscurité de ce dernier.

Etape 120 : détection d'un rayonnement rétrodiffusé (ou transmis) par l'échantillon suite à l'illumination dans chaque bande spectrale. Le photodétecteur génère un premier signal de détection $S_1$ dépendant de l'intensité du rayonnement rétrodiffusé (ou transmis) $14_1$ sous l'effet de l'illumination de l'échantillon dans la première bande spectrale $\Delta\lambda_1$ (sous-étape $120_1$) et un deuxième signal de détection $S_2$ dépendant de l'intensité du rayonnement rétrodiffusé (ou transmis) $14_2$ sous l'effet de l'illumination de l'échantillon dans la deuxième bande spectrale $\Delta\lambda_2$ (sous-étape $120_2$). Dans cet exemple, le premier signal $S_1$ et le deuxième signal $S_2$ de détection sont détectés respectivement durant l'illumination par la première source élémentaire et par la deuxième source élémentaire. Lorsqu'aucune source de lumière n'est activée, le photodétecteur acquiert un signal de bruit de fond, ou courant d'obscurité $S_B$ (sous-étape $120_3$). Ce courant d'obscurité peut être soustrait des signaux de détection $S_1$ et $S_2$.

Ainsi, comme représenté sur la figure 4B, une séquence d'éclairement $\Delta t$ dure 5ms, et est subdivisée en trois périodes temporelles de 1,66 ms, correspondant respectivement à :

- l'activation de la première source de lumière élémentaire $10_1$, et la détection, par le photodétecteur, d'un premier signal $S_1$ représentant l'intensité d'un premier rayonnement $14_1$ rétrodiffusé (ou transmis) par l'échantillon ;
- l'activation de la deuxième source de lumière élémentaire $10_2$, et la détection, par le photodétecteur, d'un deuxième signal $S_2$ représentant l'intensité d'un deuxième rayonnement $14_2$ rétrodiffusé (ou transmis) par l'échantillon ;
- la détection, par le photodétecteur, d'un signal de bruit de fond lorsqu'aucune source de lumière n'est activée.

Etape 130 : établissement d'une première fonction de détection $S_1(t)$ et d'une deuxième fonction de détection $S_2(t)$ représentant respectivement l'évolution temporelle du premier signal de détection $S_1$ et du deuxième signal de détection $S_2$. Chacune de ces fonctions est obtenue respectivement par un échantillonnage temporel du premier signal $S_1$ et du deuxième signal $S_2$, la fréquence d'échantillonnage étant par exemple de 200 Hz, ce qui correspond à une acquisition d'un premier signal $S_1$ et d'un deuxième signal $S_2$ toutes les 5 ms. L'établissement de la chaque fonction de détection peut comprendre une étape de prétraitement, de type lissage, permettant de s'affranchir d'une composante haute fréquence du signal détecté. Ce prétraitement peut prendre la forme de l'application d'un filtre passe-bas ou d'une moyenne glissante. Dans cet exemple, on effectue une moyenne glissante selon un intervalle temporel de 25 ms, soit 5 échantillons.

Etape 140 : détermination d'une première fonction dérivée $S'_1(t)$ et d'une deuxième fonction dérivée $S'_2(t)$. Chaque fonction dérivée est obtenue par une différence d'une fonction de détection à deux instants différents $t$ et $t+\delta t$. L'écart temporel $\delta t$ est de préférence inférieur à 500ms, voire à 100 ms. Dans cet exemple, $t$ et $t+\delta t$ sont des instants successifs, c'est-à-dire espacés de la période d'échantillonnage, soit 5 ms. La fonction dérivée peut être obtenue par une normalisation de la différence précédemment décrite par l'écart temporel, ce qui correspond à la définition classique d'un taux de variation.

Autrement dit,

$$S'_1(t) = S_1(t + \delta t) - S_1(t) \,(1) \text{ ou } S'_1(t) = \frac{S_1(t+\delta t) - S_1(t)}{\delta t} \,(1').$$

La deuxième fonction dérivée $S'_2(t)$ est obtenue de façon identique à la première fonction dérivée $S'_1(t)$, à partir de la deuxième fonction de détection $S_2(t)$.

Etape 150 : identification d'instants caractéristiques. Par instant caractéristique, on entend un instant auquel la fonction de détection ou sa fonction dérivée atteint une valeur particulière, franchit un seuil ou atteint un extremum local, par exemple minimum local ou maximum local. Dans cet exemple, comme décrit en lien avec les figures 2B et 3A, un instant caractéristique correspond à l'instant où une fonction dérivée franchit un seuil Th. L'étape 150 comporte alors une comparaison de chaque valeur de chaque fonction dérivée à un seuil Th, de façon à détecter un front descendant ou un front montant. Lorsque la valeur d'une fonction dérivée, par exemple la première fonction dérivée $S'_1(t)$, franchit un tel seuil, une fenêtre temporelle W est ouverte. La durée de cette fenêtre temporelle est courte, par exemple comprise entre 5 et 100 fois la période d'échantillonnage. Elle est de préférence inférieure à 500 ms et typiquement comprise entre 20 ms et 200 ms. Si, dans cette fenêtre temporelle W, l'autre fonction dérivée, en l'occurrence $S'_2(t)$ franchit également ledit seuil, le franchissement du seuil est validé pour les deux fonctions dérivées. L'instant correspondant au franchissement du seuil, par une des deux fonctions dérivée, est sélectionné comme étant un instant caractéristique $t_i$. La durée de la fenêtre temporelle W peut être prédéterminée ou ajustable.

Les figures 5A et 5B illustrent l'étape 150. Sur la figure 5A, on a représenté une première fonction de détection $S_1(t)$ et sa fonction dérivée $S'_1(t)$. Sur la figure 5B, on a également représenté une deuxième fonction de détection $S_2(t)$ et sa fonction dérivée $S'_2(t)$. La figure 5B est similaire à la figure 3A précédemment décrite. On observe un premier franchissement d'un seuil Th, par la deuxième fonction dérivée $S'_2(t)$, à $t = t_1$. Lors de ce franchissement, une fenêtre temporelle W est ouverte, de durée 100 ms. Durant cette fenêtre temporelle, aucun franchissement du seuil Th n'est détecté pour la première fonction dérivée $S'_1(t)$. L'instant caractéristique $t_1$ est donc invalidé. On observe également un deuxième franchissement du seuil Th par la première fonction dérivée $S'_1(t)$ à $t = t_2$. Lors de ce franchissement, une nouvelle fenêtre temporelle W est ouverte, au cours de laquelle le deuxième franchissement du seuil est détecté pour la deuxième fonction dérivée $S'_2(t)$. On considère alors qu'à l'instant $t_2$, les deux franchissements du seuil sont réalisés en coïncidence temporelle, autrement dit simultanément, à quelques périodes d'échantillonnages près. L'instant $t_2$ est donc sélectionné comme étant un instant caractéristique $t_i$ à considérer pour évaluer la fréquence cardiaque.

Etape 160 : détermination de la fréquence cardiaque *hr*. A partir des instants caractéristiques $t_i$ sélectionnés

lors de l'étape 150, on établit une fréquence $f$ d'occurrence des instants caractéristiques successifs, cette fréquence correspondant à la fréquence cardiaque hr. Par exemple, la fréquence d'occurrence est obtenue en effectuant une moyenne de la fréquence d'occurrence d'un nombre N d'instants caractéristiques successifs $t_i$. La fréquence $f_i$ attribuée à un instant caractéristique $t_i$ peut alors être établie selon l'écart temporel moyen entre N instants successifs précédents ledit instant caractéristique, de telle sorte que :

$$f_i = \frac{1}{\frac{1}{N-1}\sum_{j=i-N+2}^{j=i}(t_j - t_{j-1})} \quad (2)$$

La fréquence cardiaque $hr_i$ à l'instant $t_i$ est égale à $f_i$. Un changement d'unité peut alors être opéré, d'obtenir une fréquence cardiaque en $min^{-1}$.

Les figures 6A et 6B illustrent l'avantage conféré par l'invention. Sur ces figures, on a représenté une évaluation de la fréquence cardiaque d'un homme en plaçant un dispositif en rétrodiffusion, tel que représenté sur la figure 1A, au niveau du pouce. Sur la figure 6A, on a représenté une estimation de la fréquence cardiaque selon l'art antérieur, en considérant indépendamment des mesures dans la première bande spectrale et des mesures dans la deuxième bande spectrale. Sur la figure 6B, on a représenté une estimation de la fréquence cardiaque à partir d'instants caractéristiques apparaissant en coïncidence temporelle, c'est-à-dire simultanément, à la largeur de la fenêtre temporelle près, dans chaque bande spectrale. Les fonctions de détection utilisées pour établir les figures 6A et 6B sont analogues. La fréquence cardiaque déterminée en mettant en œuvre l'invention est plus stable. En particulier, les fluctuations parasites apparaissant, sur la figure 6A, à t = 80 s, t = 130 s et t = 180 s, n'apparaissent pas sur la figure 6B.

Les figures 6C et 6D sont respectivement analogues aux figures 6A et 6B, le dispositif étant disposé au niveau du poignet d'un homme. On constate également une amélioration de la précision de l'estimation lorsque l'invention est appliquée.

L'invention pourra être mise en œuvre sur un dispositif en transmission ou en rétrodiffusion porté par une personne. La configuration en rétrodiffusion est particulièrement adaptée à une intégration dans un dispositif compact de type montre, un dispositif nomade de suivi d'actimétrie ou un patch dermique. Afin d'améliorer la fiabilité de l'estimation, le dispositif sera de préférence maintenu au contact de la peau d'une personne, ou à une distance fixe de cette dernière, au moyen d'un bracelet ou autre armature rigide ou élastique.

L'invention pourra s'appliquer à la surveillance d'êtres vivants, de type nouveaux-nés, personnes âgées, sportifs ou personnes présentant un risque. L'utilisation des bandes spectrales rouge et infra-rouge sont bien adaptés pour une intégration dans dispositifs d'oxymétrie pulsée, basés sur ces mêmes bandes spectrales, de façon à déterminer d'autres paramètres physiologiques, de type saturation du sang, selon des procédés connus.

**Revendications**

1. Procédé d'estimation d'une fréquence cardiaque d'un être vivant comportant les étapes suivantes :

   a) illumination d'une zone corporelle (20) dudit être vivant par un faisceau lumineux incident (12, $12_1$, $12_2$) selon une première bande spectrale ($\Delta\lambda_1$);
   b) détection d'un rayonnement lumineux (14, $14_1$, $14_2$) transmis ou rétrodiffusé, dans ladite première bande spectrale ($\Delta\lambda_1$), par ladite zone corporelle sous l'effet de ladite illumination ;
   c) détermination d'une première fonction de détection ($S_1(t)$), représentant une évolution temporelle d'une intensité du rayonnement lumineux ainsi détecté ($S_1$);
   d) identification d'instants caractéristiques ($t_i$) à partir de ladite première fonction de détection ($S_1(t)$), et calcul d'une fréquence d'occurrence ($f$) desdits instants caractéristiques, chaque instant caractéristique correspondant à un instant auquel ladite fonction de détection atteint une valeur particulière, ou franchit un seuil, ou atteint un extremum local
   e) estimation d'une fréquence cardiaque ($hr$) à partir de ladite fréquence d'occurrence ($f$); où :

      - les étapes a) à b) sont également mises en œuvre dans une deuxième bande spectrale ($\Delta\lambda_2$), de telle sorte que l'étape c) comporte une détermination d'une deuxième fonction de détection ($S_2(t)$) représentant une évolution temporelle d'une intensité du rayonnement lumineux détecté dans la deuxième bande spectrale ($\Delta\lambda_2$);
      - l'étape d) comporte une identification d'instants caractéristiques à partir de la deuxième fonction de détection ainsi qu'une sélection d'instants caractéristiques ($t_i$), identifiés à partir de chaque fonction de détection, et apparaissant en coïncidence temporelle, la fréquence d'occurrence ($f$) étant calculée à partir des instants caractéristiques ainsi sélectionnés.

2. Procédé selon la revendication 1, dans lequel la deuxième bande spectrale ($\Delta\lambda_2$) est différente de la première bande spectrale ($\Delta\lambda_1$).

3. Procédé selon l'une quelconque des revendications précédentes, dans lequel l'étape d) comporte un calcul d'une première fonction dérivée ($S'_1(t)$) à partir de la première fonction de détection ($S_1(t)$), et d'une deuxième fonction dérivée ($S'_2(t)$) à partir de la deuxième fonction de détection ($S_2(t)$), ainsi que l'identification d'instants caractéristiques à partir de chacune desdites fonctions dérivées ($S'_1(t)$, ($S'_2(t)$)), un instant caractéristique étant un instant ou une fonction dérivée atteint une valeur particulière, ou franchit un seuil, ou atteint un extremum local.

4. Procédé selon la revendication 3, dans lequel chaque fonction dérivée est obtenue par une différence entre la valeur d'une fonction de détection en deux instants différents (t, t+$\delta$t).

5. Procédé selon l'une quelconque des revendications précédentes, dans lequel :

   - la première bande spectrale ($\Delta\lambda_1$) comporte des longueurs d'onde comprises entre 600 et 700 nm ;
   - la deuxième bande spectrale ($\Delta\lambda_2$) comporte des longueurs d'onde comprises entre 750nm et 1 $\mu$m.

6. Procédé d'estimation selon l'une quelconque des revendications précédentes, dans lequel la première bande spectrale ($\Delta\lambda_1$) s'étend entre 600 nm et 700 nm, tandis que la deuxième bande spectrale ($\Delta\lambda_2$) s'étend entre 750 nm et 1 $\mu$m.

7. Procédé selon l'une quelconque des revendications précédentes, dans lequel le rayonnement détecté est un rayonnement rétrodiffusé par ladite zone corporelle (20) sous l'effet de son illumination.

8. Dispositif pour l'estimation d'une fréquence cardiaque d'un être vivant, comportant :

   - une source de lumière (10) apte à émettre un faisceau lumineux incident ($12_1$, $12_2$) se propageant vers une zone corporelle (20) dudit être vivant, selon une première bande spectrale ($\Delta\lambda_1$) et selon une deuxième bande spectrale ($\Delta\lambda_2$) ;
   - un photodétecteur (30), apte à détecter, dans la première bande spectrale ($\Delta\lambda_1$) et dans la deuxième bande spectrale ($\Delta\lambda_2$) un rayonnement ($14_1$, $14_2$) rétrodiffusé ou transmis par ladite zone corporelle (20), sous l'effet de son illumination par ledit faisceau lumineux incident ;
   - un processeur (32), configuré pour mettre en œuvre les étapes c) à e) du procédé objet de l'une quelconque des revendications 1 à 7.

9. Dispositif selon la revendication 8, dans lequel le photodétecteur (30) est apte à détecter un rayonnement ($14_1$, $14_2$) rétrodiffusé par ladite zone corporelle (20) sous l'effet de son illumination.

10. Dispositif selon l'une quelconque des revendications 8 ou 9, dans lequel la première bande spectrale ($\Delta\lambda_1$) est différente de la deuxième bande spectrale ($\Delta\lambda_2$).

11. Dispositif selon l'une quelconque des revendications 8 à 10, dans lequel la source de lumière (10) comporte :

   - une première source de lumière élémentaire ($10_1$), apte à émettre un premier faisceau lumineux incident ($12_1$) selon la première bande spectrale ($\Delta\lambda_1$) ;
   - une deuxième source de lumière élémentaire, apte à émettre un deuxième faisceau lumineux incident ($12_2$) selon la deuxième bande spectrale ($\Delta\lambda_2$).

**Patentansprüche**

1. Verfahren zur Abschätzung einer Herzfrequenz eines Lebewesens, das die folgenden Schritte umfasst:

   a) Beleuchten eines Körperbereichs (20) des Lebewesens mit einem einfallenden Lichtstrahl (12, $12_1$, $12_2$) gemäß einem ersten Spektralband ($\Delta\lambda_1$);
   b) Detektieren einer Lichtstrahlung (14, $14_1$, $14_2$), die von dem Körperbereich unter Einwirkung der Beleuchtung in dem ersten Spektralband ($\Delta\lambda_1$) übertragen oder rückgestreut wird;

c) Bestimmen einer ersten Detektionsfunktion ($S_1(t)$), die einen zeitlichen Verlauf einer Stärke der so detektierten Lichtstrahlung ($S_1$) darstellt;

d) Identifizieren von charakteristischen Momenten (ti) anhand der ersten Detektionsfunktion ($S_1(t)$) und Berechnen einer Häufigkeit des Auftretens ($f$) der charakteristischen Momente, wobei jeder charakteristische Moment einem Moment entspricht, in dem die Detektionsfunktion einen bestimmten Wert erreicht oder einen Schwellenwert überschreitet oder ein lokales Extremum erreicht;

e) Abschätzen einer Herzfrequenz (hr) anhand der Frequenz des Auftretens ($f$); wobei

- die Schritte a) bis b) ferner in einem zweiten Spektralband ($\Delta\lambda_2$) durchgeführt werden, sodass der Schritt c) das Bestimmen einer zweiten Detektionsfunktion ($S_2(t)$) umfasst, die einen zeitlichen Verlauf einer Stärke der in dem zweiten Spektralband ($\Delta\lambda_2$) detektierten Lichtstrahlung darstellt;
- wobei der Schritt d) das Identifizieren von charakteristischen Momenten anhand der zweiten Detektionsfunktion sowie das Auswählen von charakteristischen Momenten (ti) umfasst, die anhand jeder Detektionsfunktion identifiziert werden und zeitgleich auftreten, wobei die Häufigkeit des Auftretens ($f$) anhand der so ausgewählten charakteristischen Momente berechnet wird.

2. Verfahren nach Anspruch 1, wobei sich das zweite Spektralband ($\Delta\lambda_2$) von dem ersten Spektralband ($\Delta\lambda_1$) unterscheidet.

3. Verfahren nach einem der vorhergehenden Ansprüche, wobei der Schritt d) das Berechnen einer ersten abgeleiteten Funktion ($S'_1(t)$) aus der ersten Detektionsfunktion ($S_1(t)$) und einer zweiten abgeleiteten Funktion ($S'_2(t)$) aus der zweiten Detektionsfunktion ($S_2(t)$) sowie das Identifizieren von charakteristischen Momenten anhand jeder der abgeleiteten Funktionen ($S'_1(t)$, ($S'_2(t)$)) umfasst, wobei ein charakteristischer Moment ein Moment ist, in dem eine abgeleitete Funktion einen bestimmten Wert erreicht oder einen Schwellenwert überschreitet oder ein lokales Extremum erreicht.

4. Verfahren nach Anspruch 3, wobei jede abgeleitete Funktion durch eine Differenz zwischen dem Wert einer Detektionsfunktion in zwei unterschiedlichen Momenten (t, t+$\delta$t) erhalten wird.

5. Verfahren nach einem der vorhergehenden Ansprüche, wobei:

- das erste Spektralband ($\Delta\lambda_1$) Wellenlängen zwischen 600 und 700 nm umfasst;
- das zweite Spektralband ($\Delta\lambda_2$) Wellenlängen zwischen 750 nm und 1 $\mu$m umfasst.

6. Abschätzungsverfahren nach einem der vorhergehenden Ansprüche, wobei sich das erste Spektralband ($\Delta\lambda_1$) zwischen 600 nm und 700 nm erstreckt, während sich das zweite Spektralband ($\Delta\lambda_2$) zwischen 750 nm und 1 $\mu$m erstreckt.

7. Verfahren nach einem der vorhergehenden Ansprüche, wobei die detektierte Strahlung eine von dem Körperbereich (20) unter Einwirkung der Beleuchtung rückgestreute Strahlung ist.

8. Vorrichtung zur Abschätzung einer Herzfrequenz eines Lebewesens, die Folgendes umfasst:

- eine Lichtquelle (10), die dazu fähig ist, einen einfallenden Lichtstrahl ($12_1$, $12_2$), der sich zu einem Körperbereich (20) des Lebewesens hin ausbreitet, gemäß einem ersten Spektralband ($\Delta\lambda_1$) und gemäß einem zweiten Spektralband ($\Delta\lambda_2$) zu emittieren;
- einen Photodetektor (30), der dazu fähig ist, in dem ersten Spektralband ($\Delta\lambda_1$) und in dem zweiten Spektralband ($\Delta\lambda_2$) eine von dem Körperbereich (20) unter Einwirkung seiner Beleuchtung durch den einfallenden Lichtstrahl rückgestreute oder übertragene Strahlung ($14_1$, $14_2$) zu detektieren;
- einen Prozessor (32), der dazu konfiguriert ist, die Schritte c) bis e) des Verfahrens, das Gegenstand eines der Ansprüche 1 bis 7 ist, durchzuführen.

9. Vorrichtung nach Anspruch 8, wobei der Photodetektor (30) dazu fähig ist, eine von dem Körperbereich (20) unter Einwirkung seiner Beleuchtung rückgestreute Strahlung ($14_1$, $14_2$) zu detektieren.

10. Vorrichtung nach einem der Ansprüche 8 oder 9, wobei sich das erste Spektralband ($\Delta\lambda_1$) von dem zweiten Spektralband ($\Delta\lambda_2$) unterscheidet.

**11.** Vorrichtung nach einem der Ansprüche 8 bis 10, wobei die Lichtquelle (10) Folgendes umfasst:

- eine erste elementare Lichtquelle ($10_1$), die dazu fähig ist, einen ersten einfallenden Lichtstrahl ($12_1$) gemäß dem ersten Spektralband ($\Delta\lambda_1$) zu emittieren;
- eine zweite elementare Lichtquelle, die dazu fähig ist, einen zweiten einfallenden Lichtstrahl ($12_2$) gemäß dem zweiten Spektralband ($\Delta\lambda_2$) zu emittieren.

**Claims**

**1.** Method for estimating a cardiac frequency of a living being comprising the following steps:

a) illuminating a bodily zone (20) of said living being with an incident light beam (12, $12_1$, $12_2$) in a first spectral band ($\Delta\lambda_1$);
b) detecting light radiation (14, $14_1$, $14_2$) transmitted or backscattered, in said first spectral band ($\Delta\lambda_1$), by said bodily zone under the effect of said illumination;
c) determining a first detection function ($S_1(t)$), representing a variation as a function of time of an intensity ($Si$) of the light radiation thus detected;
d) identifying characteristic instants ($ti$) from said first detection function ($S_1(t)$), and calculating an occurrence frequency ($f$) of said characteristic instants, each characteristic instant corresponding to an instant at which said detection function reaches a particular value, or crosses a threshold, or reaches a local extremum;
e) estimating a cardiac frequency ($hr$) from said occurrence frequency ($f$);

wherein:

- steps a) to b) are also implemented in a second spectral band ($\Delta\lambda_2$), such that step c) comprises determining a second detection function ($S_2(t)$) representing a variation as a function of time of an intensity of the light radiation detected in the second spectral band ($\Delta\lambda_2$);
- step d) comprises identifying characteristic instants from the second detection function and selecting characteristic instants ($t_i$), identified from each detection function, and appearing in temporal coincidence, the occurrence frequency ($f$) being calculated from the characteristic instants thus selected.

**2.** Method according to Claim 1, wherein the second spectral band ($\Delta\lambda_2$) is different from the first spectral band ($\Delta\lambda_1$).

**3.** Method according to either one of the preceding claims, wherein step d) comprises calculating a first function ($S'_1(t)$) derived from the first detection function ($S_1(t)$), and a second function ($S'_2(t)$) derived from the second detection function ($S_2(t)$), and identifying characteristic instants from each of said derived functions ($S'_1(t)$, ($S'_2(t)$)), a characteristic instant being an instant at which a derived function reaches a particular value, or crosses a threshold, or reaches a local extremum.

**4.** Method according to Claim 3, wherein each derived function is obtained via a difference between the value of a detection function at two different instants (t, t+$\delta$t).

**5.** Method according to any one of the preceding claims, wherein:

- the first spectral band ($\Delta\lambda_1$) comprises wavelengths comprised between 600 and 700 nm;
- the second spectral band ($\Delta\lambda_2$) comprises wavelengths comprised between 750 nm and 1 $\mu$m.

**6.** Estimating method according to any one of the preceding claims, wherein the first spectral band ($\Delta\lambda_1$) extends between 600 nm and 700 nm, whereas the second spectral band ($\Delta\lambda_2$) extends between 750 nm and 1 $\mu$m.

**7.** Method according to any one of the preceding claims, wherein the detected radiation is radiation backscattered by said bodily zone (20) under the effect of its illumination.

**8.** Device for estimating a cardiac frequency of a living being, comprising:

- a light source (10) able to emit an incident light beam ($12_1$, $12_2$) that propagates towards a bodily zone (20) of said living being, in a first spectral band ($\Delta\lambda_1$) and in a second spectral band ($\Delta\lambda_2$);

- a photodetector (30), able to detect, in the first spectral band ($\Delta\lambda_1$) and in the second spectral band ($\Delta\lambda_2$), radiation ($14_1$, $14_2$) backscattered or transmitted by said bodily zone (20), under the effect of its illumination by said incident light beam;
- a processor (32), configured to implement steps c) to e) of the method according to any one of Claims 1 to 7.

9. Device according to Claim 8, wherein the photodetector (30) is able to detect radiation ($14_1$, $14_2$) backscattered by said bodily zone (20) under the effect of its illumination.

10. Device according to either one of Claims 8 and 9, wherein the first spectral band ($\Delta\lambda_1$) is different from the second spectral band ($\Delta\lambda_2$).

11. Device according to any one of Claims 8 to 10, wherein the light source (10) comprises:

- a first elementary light source ($10_1$), able to emit a first incident light beam ($12_1$) in the first spectral band ($\Delta\lambda_1$);
- a second elementary light source, able to emit a second incident light beam ($12_2$) in the second spectral band ($\Delta\lambda_2$).

**Fig. 1A**

**Fig. 1B**

**Fig. 2A**

**Fig. 2B**

**Fig. 3A**

**Fig. 3B**

**Fig. 4A**

**Fig. 4B**

**Fig. 5A**

**Fig. 5B**

**Fig. 6A**

**Fig. 6B**

**Fig. 6C**

**Fig. 6D**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2355693 A
- US 9042971 B
- US 20130072771 A